# EUROPEAN PATENT APPLICATION

(11) **EP 4 134 971 A1**
(43) Date of publication of application: **15.02.2023**
(21) Application number: 21190422.2
(22) Date of filing: 09.08.2021
(51) Int. Cl.: G16H 30/20, G16H 30/40, G16H 50/20

(54) **METHOD AND DEVICES FOR SUPPORTING THE OBSERVATION OF AN ABNORMALITY IN A BODY PORTION**

(71) Applicant: Ai Medical AG, 8702 Zollikon (CH)
(72) Inventor: Federau, Christian, 8702 Zollikon (CH)
(74) Representative: Frei Patent Attorneys

(57) **Abstract**

The invention relates to the field of medical technology and concerns a method and a system for supporting a practitioner in monitoring a disease-specific anatomical location, extension and/or signal characteristic of a medical abnormality 2, in particular lesion(s), of a body portion displayed in a virtual medical presentation, for example an image, of the body portion. The method comprises an automated step S2 of identifying separate abnormalities, in particular separate abnormalities of the same kind of abnormality, in a presentation, an automated step S3 of determining, for each identified abnormality a position information indicating the position of the identified abnormality in the presentation, an automated step S4 of generating a table 4 that lists the identified abnormalities and, for each listed abnormality, a position information indicating the position of the abnormality in the body portion, and a step S5 of providing the table together with a virtual presentation 6 of the body portion to the practitioner by use of a user interface 3.

## Description

### FIELD OF THE INVENTION

The invention relates to the field of medical technology and concerns methods and devices for supporting a user, for example a practitioner, physician, radiologist etc., in monitoring a disease-specific anatomical location, extension and/or signal characteristic of one or several lesion(s) of a body portion displayed in a virtual medical presentation, for example an image, of the body portion. In particular, the invention relates to methods and devices for supporting the user in observing a disease-specific change in or on a body portion displayed in a virtual medical presentation. In particular, the change concerns an appearance, a change and/or a disappearance of an abnormality related to a medical condition. The abnormality may be a natural abnormality, this means an abnormality appearing naturally during the course of a disease, and/or a man-made abnormality, this means an abnormality caused by a medical treatment. A multiple sclerosis demyelinating lesion or a tumor are examples of a natural abnormality and an implant is an example of a man-made abnormality. The change to be observed, in particular the change of the abnormality to be observed, may concern any change observable during course of the disease.

### BACKGROUND OF THE INVENTION

Monitoring a disease-specific anatomical location, extension and/or signal characteristic, in particular monitoring a change in or on a body portion, is key to a practitioner (doctor, physician) in diagnosing a disease, assessing its evolution and its response to a specific therapy.

For example, the observation of new lesions and the change of lesions, in particular in their number or aspect, over time is crucial in the diagnosis of multiple sclerosis (MS) and in medical accompaniment of a MS patient.

Similarly, the observation of a healing process, for example after a fracture, tear or scarring, or of changes in relation to implants, for example their degradation, ingrowth or position change, is crucial for the treatment and rehabilitation of the patient.

Such observations are often done by use of medical virtual presentations of a body portion to be examined medically for example radiologic images, photographs, sonographic images, MRI-images, CT-images etc. and by an assessment of the presentation by an expert, such as a practitioner, physician, radiologist etc. Thereby, the expert may be supported by a computer.

A virtual presentation of a body portion in the sense used in this text may comprise or be a 2D image of the body portion, a 3D image of the body portion, a volume rendered image of the body portion, a spatial arrangement of such images, or a movie of the body portion, for example. A 2D image generated with a top view imaging technique (e.g. a photograph) and a slice or projection of a 3D representation of the body portion are examples of a 2D image of the body portion. A 3D visualization of the body portion or a scrollable image of the body portion are examples of a 3D image of the body portion. A movie that shows different planes of the body portion in a consecutive manner is an example of a movie presentation of the body portion.

US 2020/0294655 A1 discloses a radiology workstation that generates an attention list comprising entries that concern the actual reason for examination and entries of existing medical conditions of the patient that should be reviewed but that are not part of the actual reason for examination.

The teaching of US 2020/0380675 A1 concerns the use of Artificial Intelligence to automatize or automatize further various steps executed in or supported by computer aided detection (CAD), including automated lesion detection and identification, image co-registration, recommending treatment options and predicting the clinical outcome of the treatment options.

US 2014/0257854 A1 discloses various methods that support a practitioner in analyzing and reporting medical images. According to an aspect, a method for providing medical diagnostic images is provided in which, with the aid of a processor, it is determined whether each of a plurality of medical images is of medical interest to a reviewing physician. Further, a representative for the set of images that have been determined to be of interest to a reviewing physician may be provided. The set of images and its representation may be used for supporting the physician in preparing a report. According to another aspect disclosed in US 2014/0257854 A1, a method for providing patient medical diagnostic images is provided. The method comprises retrieving historical data of the patient from a memory, comparing, using a processor, one or more current images associated with the patient with the historical data, and providing the one or more images and historical data to a reviewing physician. The method may comprise a step of comparing the patient's current images to the patient's historic images. The method may comprise comparing initial findings to outcomes recorded in historical data, and making that additional finding available to the physician. Yet a further aspect disclosed in US 2014/0257854 A1 concerns a method that provides an alert for an urgent medical condition if one or more image falls within one or more boundary parameters stored in a memory.

US 2021/011775 A1 discloses a method for classifying, or otherwise diagnosing, and measuring aneurysms and their change over time in an automated manner. The method includes accessing medical image data that depict vasculature in a subject and generating a binary vasculature mask by segmenting the medical image, wherein the binary vasculature mask indicates the absence or presence of vasculature at a voxel location. The binary vasculature mask is used as input to a trained machine learning algorithm for generating classified feature data, wherein the classified feature data classify regions in the vasculature of the subject as being associated with an aneurysm. A probability map is generated from the classified feature data, wherein the probability map indicates a probability of locations in the vasculature of the subject being associated with an aneurysm. Finally, quantitative parameters are computed by fitting a basis set of geometrical objects to values in the probability map, wherein the quantitative parameters quantify aneurysm geometry. The quantitative parameters include parameters of said geometrical objects, such as volume, orientation and dimension, and they can be used to measure and report the size and location of the aneurysms. The quantitative parameters are used for generating, in an automated manner, a report that indicates a quantitative analysis of one or more aneurysms in the vasculature of the subject.

In an embodiment, the method according to US 2021/011775 A1 is part of a method for tracking changes in an aneurysm over time. Therefore, the binary vasculature mask generated for a current image is co-registered with a binary vasculature mask generated for a previous image and the quantitative parameters generated therefrom are compared.

All steps of the methods disclosed in US 2021/011775 A1 are carried out in an automated manner, this means by use of a computer system. By this, the purpose of the method for tracking changes according to US 2021/011775 A1 is fulfilled, namely to be a method for tracking changes that removes a level of subjectivity and that provides an objective measure of changes is fulfilled.

Various documents disclose methods and systems that supports the practitioner in issues related with the constantly increasing number of medical images taken for a specific patient, wherein the images may differ in the body portion shown, in the size of the body portion shown, in the viewing direction, and in the facility used or the settings of the facility used, for example. WO 2014/030092 A1, US 10304564 B1 and US 2020/0043167 A1 are examples of such documents.

WO 2014/030092 A1 discloses an annotation support system and a method for providing annotation support that help to identify and display medical images of a prior image study that match with a medical image of a current image study. Therefore, a context of the current medical image, for example the current patient, a modality of the current image study or an anatomy, is determined automatically and compared to the contexts of the medical images of the prior image study.

US 10304564 B1 discloses methods and systems in which a selection of a plurality of medical images is displayed to a user in a specific chronological order based on a classification determined automatically for each of the plurality of medical images and/or on a set of rules determined automatically.

US 2020/0043167 A1 discloses a method and apparatus for automatically creating a comparison layout in a graphical user interface (GUI), wherein the comparison layout comprises at least one medical image from an image series of a current study adjacent to at least one medical image from an image series of a previously-created study. For identifying the images to be presented in the comparison layout, a processor performs an image recognition routine and a comparison between at least one image series of the current study and at least one image series of the previously-created study, wherein the comparison comprises calculating image similarity between the at least one image series of the current study and the at least one image series of the previously-created study.

Although the state-of-the-art provides various approaches for supporting a practitioner in assessing medical presentations, the manner the practitioner is supported in completing his/her challenging task under time pressure can be further improved.

For example, while automated methods may in general be useful to increase the speed of the workflow of some tasks, their reliability are typically insufficient in the context of medical decisions where a zero-error tolerance should be applied. In particular, automated methods according to the state-of-the-art are not powerful enough to assess in error free manner medical presentations such as magnetic resonance images which are heterogeneous and with complex signal properties, involving many artefacts that could mimics some diseases.

Therefore, the experience and risk tolerance of a practitioner (usually a specialist/expert) are key in assessing medical presentations in many cases and the interpretation of findings necessitates the incorporation of conceptual information by the practitioner. For example, some degree of brain atrophy may be interpreted as a normal finding in an aged patient, but may be an alarming sign of a serious disease in a younger patient.

For these reasons, assessments of often complex medical conditions that are generated in an automated manner, said manner providing to a user no or only cumbersome corrections possibilities, are of limited use in practice. State-of-the-art methods and devices ignore often this issue or just accept it. Therefore, there is need to improve the interface between automated steps of the assessment and steps that the user (practitioner) wants or has to carry out.

Further, it is important that a specific user (practitioner) gets the information she/he needs for an assessment in a manner that she/he wants and that she/he can manipulate this information in a straight forward manner. This could not only improve the workflow, but also reduce the number of errors. Indeed, by reducing the effort necessary to perform a correction step, the attention of the practitioner is kept high.

Even further, the support of the practitioner may be improved further with respect to balancing better what is done in an automated manner and what is done by the practitioner. A support with a high degree of automatization, as disclosed in US 2021/011775 A1 for example, is not what a practitioner always wants. Rather, it is important that the practitioner leads the assessment at each stage. Any need for cumbersome corrections of automatically generated assessments does not support the practitioner.

### SUMMARY OF THE INVENTION

It is an object of the invention to provide methods and devices that overcome at least some of the drawback of methods and devices according to the state-of-the-art. In particular, it is an object of the invention to provide a method and a system for supporting a user in observing an abnormality in or on a body portion, wherein the body portion is displayed in a virtual medical presentation and wherein the method and system overcomes at least some of the drawbacks of methods and devices according to the state-of-the-art.

In particular, it is an object of the invention to provide a method and a device that displays information contained in current virtual medical presentation, and usually in a virtual previous medical presentation, of the body portion in an ergonomic manner, this means in an ordered, in particular list-based, manner that allows the practitioner to identify, evaluate and/or compare medical conditions, for example findings concerning the evolution of an anatomical extension and signal characteristics of one or several lesion(s), in the most efficient way.

It is a further object of the invention to provide a method and a device for producing a practitioner-derived, this means practitioner-corrected and practitioner-validated, as opposed to purely automated, quantitative list, this means table, of findings (also called abnormalities in this text). The table, and hence the findings, may be editable in a user-friendly manner. In particular, each finding related to an entry may be studied and corrected individually. The practitioner-derived table may be translated in an automatic manner in a report. Therefore, it is yet a further object of the invention to provide a method and a device for generating a practitioner-derived report in an automated manner.

The method is a computer-implemented method or a computer supported method at least. Therefore, it is a further object of the invention to provide a related computer program, a related computer readable medium, a related computer-readable signal, and a related data carrier signal.

At least one of the objects is achieved by the invention described in the following and/or the invention claimed in the claims.

The term "virtual presentation" (in short "presentation") has the meaning as pointed out above unless otherwise specified.

Further, the virtual presentations used in the method are usually co-registered virtual presentations. This means that the presentations have been or are transformed in a manner that the similarity of the presentations is maximized with respect to the appearance of a reference presentation and/or with respect to one another.

For example, the presentations are coregistered to a reference presentation, such as a template presentation, wherein the template presentation can be one of the presentations generated for the body portion, and hence to one another, or the presentations are coregistered directly to one another. Coregistration might improve the ease to compare presentations.

Co-registration may be carried out with respect to presentation ("image") orientation, presentation position (image position, slice number), position and orientation of the body portion shown, zoom, presentation ("image") contrast etc.

For example, coregistration may comprise a step of transforming the presentation to the template presentation, and a step of resampling the transformed presentation to the template presentation, for example using interpolation, so that the voxel positions of the transformed presentation match the voxel positions of the template presentation.

A method according to the invention is a method for supporting the observation of a medical abnormality in or on a body portion displayed in a virtual medical presentation of the body portion ("presentation" in the following).

The observation may comprise at least one of observing an appearance, a change, and a disappearance of the abnormality. In addition or alternatively, the observation may comprise at least one of determining the number of abnormalities, a location of the abnormalities determined, a volume of the abnormalities determined and/or a change in at least one of the number, location and volume of the abnormalities determined.

The abnormality may be a natural abnormality or a man-made abnormality as mentioned above.

Usually, it is not only one presentation that is considered in the method but a plurality of presentations belonging to a study, for example a radiologic, MRI, CT, sonographic, or photographic study. In embodiments, in particular in embodiments aiming for observing a change of the body portion over time, in particular a change with respect to an abnormality, a plurality, this means at least two studies, are considered in the method.

Any presentation or study used in any embodiment of the method may be received from the imaging system used for generating the presentation or study or from a server/archive on or in which the presentation or study is stored, such as a picture archiving communication system (PACS), for example.

Any presentation or study used in any embodiment of the method may be taken according to a given procedure and/or stored in an independent format, such as the so-called DICOM format.

The method comprises the following steps:
- A step of screening a virtual current presentation of a current study of the body portion for the abnormality, a step of identifying separate abnormalities, if more than one of the abnormality is shown in the current presentation, and a step of determining, for each identified abnormality, a position information that indicates the position of the identified abnormality in the current presentation.
   The steps of screening, identifying and determining are automated steps or semi-automated steps at least. In particular, the steps are carried out without any support by a user, except an optional initiation of at least one step or at least one substeps and/or an optional correction step by the user.
   In other words, the separate (individual) abnormalities of a specific kind of abnormality are identified and a position information for each separate (individual) abnormality of the specific kind of abnormality is determined in a higly automated manner.
   For example, an overall (global) extension of abnormality on voxel (pixel, in the case of a 2D presentation) level may be determined in the step of screening. In particular, the voxels (pixels) may be determined that have a high probability of showing the abnormality, for example by having a probability-value for showing the abnormality that is higher than a minimal probability value (threshold) and optionally lower than a maximal probability value.
   The step of identifying separate abnormalities may then identify non continuous sets of voxels (pixels) of the determined overall (global) extension of abnormality.
   The non continous sets of voxels may be considered as representing separate abnormalities and the position information indicating the position of a separate abnormality may correspond to an identifier for the location of the set of voxels (pixels) in the presentation, wherein said set of voxels (pixels) represents the separate abnormality.
- A step of generating a table (list).
   The step of generating a table is an automated step, except an optional initiation by the user.
   The generated table lists the identified abnormalities and, for each listed abnormality, a position information indicating the position of the abnormality in the body portion.
   In other words, the table comprises an entry, in particular a row or column of entries, for each separate abnormality that has been identified, wherein each entry comprises a position information indicating the position of the corresponding abnormality in the body portion.
   The position information indicating the position of a separate abnormality in the body portion may be any information indicating the anatomical location of the separate abnormality. Therefore, the position information indicating the position of the separate abnormality in the body portion can be considered as being different from any position information indicating the position of the separate abnormality in a presentation that, as pointed out above, is indicative for the location of the set of voxels (pixels) representing the separate abnormality in the presentation.
- A step of providing the table and a virtual presentation of the body portion, wherein the virtual presentation is a virtual presentation of the current study, to a user via a user interface. The user interface comprises therefore an image viewer and the virtual presentation is displayed in the image viewer.
   The presentation provided to the user may be the current presentation.

As pointed out above, the table comprises usually an entry for each identified abnormality, wherein the entry may be a row or column of the table, wherein the row or column comprising a plurality of entries that relate to the corresponding abnormality. In other words, the term "entry" may relate to a single entry in the table or to a row or column of entries.

Independent of the concrete realization of the method and its purpose, at least one of the following may apply to the table generated in the step of generating a table:
- The table may be editable by the user. For example,
   ∘ each entry of the table may be editable independently of any other entry of the table;
   ∘ the user may add a new entry to the table and/or remove an entry from the table.
- Each entry of the table may be linked to at least one presentation on which the abnormality related to the entry is visible.
   In addition, the table may be configured to display a presentation on which the abnormality related to the entry is visible in the image viewer if the user activates the entry.
   Activation of the table may cause highlighting of the listed abnormalities that are visible in the presentation displayed in the image viewer. The abnormalities may be highlighted in the presentation and/or in the list.
   Activation of an entry may cause highlighting of the abnormality related to the activated entry in the presentation displayed in the image viewer.
   In an embodiment, an entry may be linked to a plurality of presentations in which the abnormality related to the entry is visible and/or it may be configured to change the manner the presentation displayed in the image viewer is presented, wherein the presentations or the manner of presentation differ in their viewing angle and/or in the plane of the body portion shown, for example.
   In addition, the entry may be configured to select a specific presentation of the plurality of presentations to which the entry is linked, said specific presentation being then displayed in the image viewer, and/or to change the manner the presentation is displayed in the image viewer is presented.
   The entry may comprise a control element for selecting the specific presentation and/or the specific manner the presentation displayed in the image viewer is presented. Alternatively or in addition, keyboard shortcuts may be used, for example.
   The control element may be a drop-down list comprising, for example, the available viewing angles and/or a drop-down list comprising the available planes.
   The control element may be a button configured to browse through, for example, the available viewing angles and/or a button configured to browse through the available planes.
   In particular, browsing may base on one-click operations.
- The table may list, for each listed abnormality, further information besides the position information. For example, the table may list at least one of:
   ∘ The anatomical extent of the abnormality.
   ∘ The anatomical aspect of the abnormality.
   ∘ The signal characteristic of the abnormality.
   ∘ Whether the abnormality is observed for the first time or not. In other words, the table comprises an evolution information.
   ∘ The volume of the abnormality.
   ∘ Information concerning contrast uptake.
   ∘ Information derived from at least one of the above-listed information.
      For example, information related to a classification of the abnormality may be derived and listed. If the abnormality is a multiple sclerosis lesion for example, the information may describe a variable of the McDonald criteria where multiple sclerosis lesions are classified in four categories depending on their anatomical placement, namely juxtacortical, periventricular, infratentorial, and spinal.
      Information that is related to a classification depending on the anatomical placement of the abnormality is called "placement" in the following.
      The information may be derived from the anatomical localization, anatomical extend, anatomical aspect and/or contrast update, for example.

   In an embodiment, the table may be an active table in the meaning that information related to an abnormality of a new entry that is added to the table by the user and/or to an entry corrected by the user is generated and linked to the new entry in an automated manner.
   For example, the user may add an entry for an abnormality that is not listed in the list so far and indicate the position, and optionally the extent, of the abnormality. Then, at least one of the position information indicating the position of the abnormality in the current presentation, the position information indicating the position of the abnormality in the body portion, the evolution information, the volume of the abnormality, the placement of the abnormality, information concerning contrast uptake, and presentations that are or may be relevant for the abnormality is determined in an automated manner.
   The same may apply to an entry that is corrected by the user, for example by indicating a corrected extension and/or corrected position.

In an embodiment, the method is a method for observing a change of the body portion over time, in particular a change with respect to an abnormality.

In this embodiment, the method comprises the step of screening a current presentation for the abnormality in any embodiment disclosed, the step of identifying separate abnormalities in any embodiment disclosed, the step of determining, for each identified abnormality, a position information in any embodiment disclosed, the step of generating a table in any embodiment disclosed, and the step of providing the table and a virtual presentation of the current study of the body portion to a user via a user interface in any embodiment disclosed.

In this embodiment, the method comprises further a step of determining for each identified abnormality whether there is a related abnormality in a virtual previous presentation of a previous study of the body portion, and the table generated in an automated manner indicates, for each listed abnormality, whether there is a related abnormality in the previous presentation or not. In other words, the table may list, for each listed abnormality, the evolution information mentioned above. The evolution information may be a binary information indicating whether the abnormality is new/newly observed or old/previously observed.

The step of determing for each identified abnormality whether there is a related abnormality in a previous presentation of the body portion may comprise determining, using the position information indicating the position of the identified abnormality in the current presentation, whether there is an abnormality in the previous presentation having a position information indicating that the abnormality in the previous presentation corresponds to the abnormality identified in the current presentation, for example.

In particular, a version of the previous presentation that is co-registered with the (usually co-registered) version of the current presentation is or was screened for the abnormality and a position information indicating the position of the identfied abnormality in the previous presentation is or was determined for each identified abnormality. The amount of position information generated in this manner for the previous presentation is screened then for a corresponding position information of the amount of position information determined for the current presentation.

In many embodiments, the previous presentation is a former current presentation and the position information indicating the position of the identified abnormality and further, optional as the case may be, features related to the previous presentation were determined (generated, amended, modified etc.) during carrying out the method, wherein the previous presentation was the current presentation.

The same is true for other presentations and related features, mentioned in this text ("second last", third last..., for example).

In an embodiment of the method for observing a change of the body portion over time, the step of determining, for each identified abnormality, whether there is a related abnormality in a previous presentation of the body portion may comprise:
- An automated step of generating a current data set. The current data set comprises, for each identified abnormality, a current data element that comprises the position information indicating the position of the identified abnormality in the current presentation.
   The current data set may comprise or be a table comprising, for each identified abnormality, a current data element given by a row or column of the table. However, the current data set may be given by any element and/or algorithm suitable to gather the information given by the current data set.
   Other data sets, for example the previous data set, and other data elements, for example the previous data element, are usually of the same kind as the current data set and the current data element.
- An automated step of receiving a previous data set of the previous presentation of the body portion. The previous data set comprises, for each abnormality identified in the previous presentation, a previous data element that comprises a position information indicating the position of the abnormality identified in the previous presentation.
   As mentioned, the previous presentation, the related position information and the previous data set may be a former current presentation, the position information related to the former current presentation, and a former current data set. Therefore, they may have been generated as disclosed with respect to the current presentation, the position information related to the the current presentation, and the current data set.
- An automated step of screening, for each current data element, the previous data set for a previous data element comprising the abnormality at a position in the previous presentation that corresponds to the position of the abnormality according to the current data element.
   Screening for each current data element the previous data set for a previous data element comprising the abnormality at a position in the previous presentation that corresponds to the position of the abnormality according to the current data element may comprise determining whether the voxels (pixels) at which an abnormality was identified in the the privious presentation are within a preset range comprising the voxels (pixels) at which an abnormality was identified in the current presentation.
- Optionally, the method may comprise further an automated step of setting an evolution tag for or in each current data element for which a previous data element comprising the abnormality at a position in the previous presentation that corresponds to the position of the abnormality according to the current data element was found.
   In other words, the evolution information may be stored, for each identified abnormality, in a simple manner.
   In embodiments in which the table lists evolution information, the listed evolution information may rely on the stored evolution information, in particular on the set evolution tag.

In an embodiment, the abnormalities identified in the current study as well as the abnormalities identified in the previous study or previous studies but not in the current study are listed in the table. In other words, all abnormalities identified for the body portion of the patient may be listed.

Optionally, the entry related to an abnormality may indicate when or in which study the abnormality was observed for the first time and/or for the last time.

In an embodiment of the method, the method comprises the following steps:
- A step of providing a list of studies comprising a first item that is characteristic for the current study and a second item that is characteristic for the previous study.
   For example, the first item may comprise a description defining the current study and the second item may comprise a description defining the previous study.
   For example, the description may be the date of a corresponding study.
   In an embodiment, the list of studies is provided automatically by reading the study date upon reception of the study or a presentation of the study.
- A step of providing a list of presentations, comprising at least one item that is characteristic for a kind of presentation.
   For example, each item may comprise a description defining a specific presentation.
   Usually, a first kind of presentation differs from a second kind of presentation in the settings used during acquisition of the presentations or raw data and/or features used during processing of the raw data. An item, for example its description or labeling, may then comprise a hint towards the settings and/or features used for the kind of presentation to which the item refers.
   In particular, the items, more precisely their labeling, may name or indicate the available or used settings and/or features. In the case of CT and MRI examination, the label of the item may comprise terms such as FLAIR or T1-weighted for MRI, or soft-tissue or bone window for CT.
   The list of presentations, in particular if it is a list of presentations that is appropriate for a specific type of study and/or a specific medical case, can be provided in an automated manner or it can be defined by the user.
   In an embodiment, the list of presentations is provided in an automated manner, for example by reading out meta data.
- A step of assigning the current study, or a subset thereof, to the item of the list of studies that is characteristic for the current study and assigning the previous study to the item of the list of studies that is characteristic for the previous study.
   The assignment of studies to the list of studies is such that maximal one study is assigned to each item of the list of studies. Typically, one study is assigned to every item of the list of studies.
- A step of assigning a presentation of the current study to the item of the at least one item of the list of presentation that is characteristic for the presentation of the current study and a presentation of the previous study to the item of the at least one item of the list of presentations that is characteristic for the presentation of the previous study.
   The assignment of presentations to the lists of presentations (to the lists of presentations if more than one list of presentations is provided, for example one for each study assigned to an item in the list of studies) is such that maximal one presentation is assigned to each item of the (each) list of presentations. However, there may be one or more items to which no presentation is assigned in cases in which no assignment is possible, for example because a presentation of the kind to which an item relates has not been generated.
   In summary, the current and previous (more general all) presentations available for a patient can be assigned to one of the items of the list of studies or to none by assigning or not assigning the study to which a presentation belongs to an item of the list of studies, and to one of the items of a list of presentations or to none by assigning or not assigning the presentation to an item of the list of presentations.
   In an embodiment, a mechanism is provided to ensure that maximal one study of the patient is assigned to an item of the list of studies and that maximal one presentation is assigned to an item of the list of presentations. An example of such a mechanism is if there is more than one presentation that could be assigned to one item of a list of presentations, then the presentation with the later date is assigned.
   In an embodiment, a set of rules is used in the step of assigning a study to an item of the list of studies and/or in the step of assigning a presentation to an item of the list of presentations.
   For example, a list of characteristics, this means of kinds of studies and/or of kinds of presentations, that are to be assigned (list of including characteristics in the following) can be provided, for example by the user.
   The method according to this embodiment can comprise a step of scanning the name of a study and/or of a presentation or scanning other metadata related to the study and/or presentation for a characteristic, in particular for a characteristic comprised in the list of including characteristics.
   The assignment of the study or presentation can then depend on whether the scanned name or metadata comprises at least one characteristic that corresponds to at least one characteristic of the list of including characteristics.
   Alternatively or in addition, a list of characteristics, this means of kinds of studies and/or of kinds of presentations, that are not to be assigned (list of excluding characteristics in the following) can be provided and used in the step of assigning a study and/or in the step of assigning a presentation.
   In embodiments, the characteristics of the list of including characteristics and/or of the list of excluding characteristics as well as the characteristic or characteristics for which the name or metadata is scanned are given as terms, this means as a sequence of letters and/or numbers. In other words, the list of including characteristics may be a list of including terms and the list of excluding characteristics may be a list of excluding term.
   In an embodiment, the scanned name or metadata must comprise any term comprised in the list of including terms or a subset thereof for the study or presentation related to said name or metadata being assigned.
   In an embodiment, a plurality of lists of including terms is provided and the scanned name or metadata must comprise, for each of the provided lists of including terms, at least one term that is comprised in the list of including terms for the study or presentation related to said name or metadata being assigned.
   For example, the assignment can be determined by scanning the name, for example the sequence name in the case of MR examination, of the presentation, said name is normally saved in the header of the presentation, and by assigning the presentation if a term found during scanning corresponds to an item of a predefined list of including terms, while at the same time the term found corresponds to none of the items of a list of excluding terms.
   Alternatively or in addition, a model of an artificial intelligence (AI), such as a neural network, may be used in the step of assigning a study to an item of the list of studies and/or in the step of assigning a presentation to an item of the list of presentations.
   In particular, the model of the AI may be trained to classify a presentation according to the items of the list of presentations. For example, the model may be trained to recognize the contrast of the presentation.
   In an embodiment, the method comprises a step of correcting the assignments done according to any embodiment disclosed by the user. Therefore, the user interface may comprise tools configured for correcting assignments by the user, for example a pop-up view, such as a preference panel.
- A step of providing the list of studies or an adapted list of studies and the list of presentations or an adapted list of presentations to a user via the user interface, wherein the user interface comprises a first view for the list of studies or the adapted list of studies and a second view for the list of presentations or the adapted list of presentations.
   The adapted list of studies may comprise only the items of the list of studies to which a study was assigned and/or it may comprise only items related to studies selected by the user. In addition or alternatively, the adapted list of studies may be organized (ordered) differently than the list of studies.
   The adapted list of presentations may comprises only the items of the list of presentations to which a presentation was assigned and/or it may comprise only items related to kind of presentations selected by the user. In addition or alternatively, the adapted list of presentations may be organized (ordered) differently than the list of presentations.
   The first view and the second view are configured to allow the selection of an item of the list shown in the first view and the list shown in the second view, respectively.
   The first view, the second view and the image viewer are configured for the presentation to be displayed in the image viewer that belongs to the study and kind of presentation indicated by the item selected in the list displayed in the first view and the item selected in the list displayed in the second view.
- A step of selecting a presentation to be displayed in the image viewer by selecting an item of the list shown in the first view and by selecting an item of the list shown in the second view.
   The selected presentation to be displayed is displayed immediately after selection and in an automated manner, usually.
   Usually, the items of the list shown in the second view are linked to the assigned presentations of the study that is assigned to the selected item of the list shown in the first view.
   This also means that the presentation to which an item of the list shown in the second view refers depends on the item selected in the list shown in the first view. This is in particular the case if the item of the list shown in the second view refers to a kind of presentation that is present in a first study assigned to a first item of the list shown in the first view and in a second study assigned to a second item of the list shown in the in the first view.
   Further, this means that the presentation to which an item of the list shown in the second view refers changes if the item selected in the list shown in the first view changes.

Details of a method and a system for selecting, and - as the case may be - displaying, ordering and/or navigating, virtual presentations of a body portion by use of a list of studies and a list of presentations, is disclosed in the European Patent Application having the application number EP 21190411. EP 21190411 is enclosed herewith by reference.

In an embodiment, the step of assigning studies, such as the current study and the previous study, to items of the list of studies, the step of assigning presentations to items of the lists of presentations, and the step of providing to a user the (adapted, as the case may be) list of study and the (adapted, as the case may be) list of presentations are automated steps. This means these steps are carried out in an automated manner, except an optional initialization by the user.

Optionally, the step of providing a list of studies and/or the step of providing a list of presentations may be an automated step.

In an embodiment, the entries of table that correspond to abnormalities that may be observed in the selected and displayed presentation may be highlighted.

In an embodiment, the anatomical location of an abnormality selected by selecting the corresponding entry in the table may be highlighted in the selected and displayed presentation.

Due to the fact that the presentation displayed in the image viewer belongs to the study and kind of presentation indicated by the item selected in the list displayed in the first view and the item selected in the list displayed in the second view, a change in the item selected in the list displayed in the first view, this means in the (adapted, as the case may be) list of studies, causes a change of the presentation displayed in the image viewer to a presentation belonging to the newly selected study and being of the same kind, this means having the same characteristic with respect to the characteristics indicated by the items of the list shown in the second view, this means the (adapted, as the case may be) list of presentations, (no change of the item selected in the list shown in the second view) as the presentation displayed before changing the item selected in the list shown in the first view.

Due to the fact that the presentation displayed in the image viewer belongs to the study and kind of presentation indicated by the item selected in the list displayed in the first view and the item selected in the list displayed in the second view, a change in the item selected in the list displayed in the second view, this means the (adapted, as the case may be) list of presentations, causes a change of the presentation displayed in the image viewer to a presentation of the newly selected kind of presentation and belonging to the same study (no change of the item selected in the list shown in the first view) as the presentation displayed before changing the item selected in the list shown in the second view.

Alternatively, or in addition, to selecting an item of the list shown in the first view and/or of the list shown in the second view by clicking on the item, the user interface may comprise a toolbar item configured to navigate in the list shown in the first view and to change the currently displayed study. In particular, the toolbar item may be configured to move up and/or down in the list shown in the first view.

Similarly, the user interface may comprise a toolbar item configured to navigate in the list shown in the second view and to change the currently displayed kind of presentation. In particular, the toolbar item may be configured to move up and/or down in the list shown in the second view.

Alternatively, or in addition, a system on which the method is carried out may be configured to accept one or more keyboard shortcuts that permit to navigate in the list shown in the first view and/or the list shown in the second view and to change the currently selected item of the list shown in the first view and/or of the list shown in the second view. In particular, the system may be configured to accept one or more keyboard shortcuts for moving up and/or down in the list shown in the first view and/or the list shown in the second view.

In an embodiment, the method comprises at least one of the following steps carried out by the user, in particular by the user interface comprising tools allowing the user to carry out at least one of the following steps:
- A step of replacing the presentation displayed in the image viewer with a replacement presentation that corresponds to a presentation assigned to another item of the list shown in the second view but to the same item of the list shown in the first view.
   The step of replacing may comprise activating the item to which the replacement presentation was assigned by clicking on the item, by pressing a toolbar item, or by using a keyboard shortcut, for example.
   In particular, the replacement presentation corresponds to the presentation assigned to the item arranged in the list shown in the second view precedingly or subsequently to the item to which the presentation displayed in the image viewer is assigned.
   In particular, the replacement presentation corresponds to a presentation assigned to the item arranged in the list shown in the second view precedingly or subsequently to the item to which the presentation to which the presentation displayed in the image viewer is assigned.
   In other words, the user can initiate a replacement of the presentation displayed in the image viewer with a replacement presentation that is a presentation of the same study as the presentation to be replaced (this means the presentation currently displayed in the image viewer). The replacement presentation can be assigned to the item that is arranged in the list shown in the second view one position above or one position below the item to which the presentation to be replaced is assigned. This also means that the sequence of replacements may occur in a predefined manner if the list shown in the second view looks always the same.
- A step of replacing the presentation displayed in the image viewer with a replacement presentation that corresponds to a presentation of a study assigned to another item of the list shown in the first view but to the same item of the list shown in the second view.
   The step of replacing may comprise activating the item to which the replacement presentation was assigned by clicking on the item, by pressing a toolbar item, or by using a keyboard shortcut, for example.
   In particular, the replacement presentation corresponds to a presentation of the study assigned to the item arranged in the list shown in the first view precedingly or subsequently to the item to which the study to which the presentation displayed in the image viewer is assigned.
   In other words, the user can initiate a replacement of the presentation displayed in the image viewer with a replacement presentation that is a presentation of the same kind as the presentation to be replaced (this means the presentation currently displayed in the image viewer). The replacement presentation can be assigned to the item that is arranged in the list shown in the first view one position above or one position below the item to which the presentation to be replaced belongs. This also means that the sequence of replacements may occurs in a predefined manner if the list shown in the first view is an ordered always in the same manner.
- A step of switching back and forth between displaying in the image viewer a first presentation and a second presentation, wherein the first presentation is a presentation assigned to a first item in the list shown in the second view and the second presentation is a presentation assigned to a second item in the list shown in the second view. The first presentation and the second presentation are usually assigned to the same item of the list shown in the first view, this means they belong to the same study.
   The step of switching back and forth may comprise activating items by clicking on the item, by pressing a toolbar item, or by using a keyboard shortcut, for example.
- A step of switching back and forth between displaying in the image viewer a first presentation and a second presentation, wherein the first presentation is a presentation of the study assigned to a first item in the list shown in the first view and the second presentatin is a presentation of the study assigned to a second item in the list shown in the first view. The first presentation and the second presentation are usually assigned to the same item of the list shown in the second view, this means they are of the same kind of presentations.
   The step of switching back and forth may comprise activating items by clicking on the item, by pressing a toolbar item, or by using a keyboard shortcut, for example.

A tool, such as a toolbar item of the user interface or a keyboard shortcut, may be available in the step of switching back and forth according to the above third or forth bullet point. The tool may be configured to perform the replacement of the presentation displayed in image viewer in a toogle way, this means a replacement of the displayed presentation when using the tool and a replacement back to the initially displayed presentation when using the tool again.

Such a "toogle tool" may be used for example to switch back and forth between the current study and the immediate previous study to compare and analyse if a region of interest, such as a medical lesion, is modified in the current study compared to the previous study.

Alternatively or in addition, such a "toogle tool" may be used to switch back and forth between presentation belonging to the same study but being of a different kind, for example having different contrast, to assess for example a region of interest or a medical lesion in different contrasts. An important example is, while assessing a medical lesion on the FLAIR contrast, to switch to a contrast-enhanced contrast to verify if the medical lesion uptakes contrast or not, and switch back to the FLAIR contrast to continue the examination of the rest of the presentations.

In an embodiment, the step of screening the current presentation for the abnormality, comprises a step of running a model of an artificial intelligence (AI), said model being trained for localizing the abnormality in a presentation of the body portion.

The model may be trained for segmenting the presentation in voxels (pixels) comprising the abnormality and in voxels (pixels) not comprising the abnormality.

In an embodiment, the model is trained for segmenting the presentation. In this case, an output of the step of running the model may comprise a probability map, wherein the probability map indicates the probability of each voxel (pixel) to contain the abnormality.

In other words, the model is trained for assigning a probability to each voxel of the current presentation that the voxel to belongs to the abnormality, for example by the step of screening, comprising a step of generating a probability map.

The abnormality is then determined as the voxels which have a probability that is comprised within the range between a minimal probability-value and a maximal probability-value.

The minimal probability-value and/or a maximal probability-value can be modified by the user, in particular for correcting an abnormality identified in an automated manner.

The probability of each voxel (pixel) to show the abnormality may be given by a probability-value of a range of probability-values, said range of probability-values starting at a bottom probability-value and ending at a top probability-value. The bottom probability-value may indicate that the abnormality is not visible (probability of 0 or 0 %) or most likely not visible and the top probability-value may indicate that the abnormality is visible (probability of 1 or 100 %) or most likely visible.

For example, the range of probability-values may be 0 to 1, or 0 to 10, or 0 to 100 or the like.

In an embodiment, a plurality of models of the artificial intelligence and/or a model of a first artificial intelligence and a model of a second artificial intelligence are run. The models run are trained for localizing the abnormality in a presentation of the body portion, in particular for segmenting the presentation.

In particular, the models run are trained for assigning a probability to each voxel of the presentation that the voxel belongs to the abnormality, wherein, for each model run, a probability segmentation map is generated.

If a plurality of models and/or models of different AIs are run, the user may select an output of one of the models, for example a generated probability segmentation map.

The method, in any embodiment disclosed, may provide opportunities, for example using the quantitative correction area disclosed with respect to the system, to the user to amend the findings of the automated steps and/or to amend the automated steps. For doing so, at least one of the following may apply, for example:
- The method comprises a step of correcting by the user the extend and/or position of an identified abnormality by at least one of the following:
   ∘ By modifying at least one of a minimal probability-value that is considered in the step of screening the current presentation for the abnormality and a maximal probability-value that is considered in the step of screening the current presentation for the abnormality.
      In particular, the minimal probability-value and the maximal probability are the probability values defining the range for determining whether a voxel shows the abnormality or not.
   ∘ By directly adding voxels to or removing voxels from a set of voxels representing an abnormality listed in the table.
      "Directly" means that the voxels are added or removed on voxel-level, using a selector directly on the presentation, which defines which voxel should be added or removed.
      The set of voxels may be the set of voxels disclosed with respect to the position information indicating the position of a separate abnormality in a presentation, for example the current presentation.
      Optionally, the voxels that may be added are restricted to a plurality of voxels (called "underlying voxels" in the following), wherein the plurality of voxels is determined in an automated manner.
      The underlying voxels can be determined by one or by one of several automatic method(s), which can possibly be selected by the user.
      An example of such an automatic method is a machine learning model, trained for assigning a probability to each voxel of a presentation of the body portion, for example of the current presentation, that the voxel belongs to the voxels representing the abnormality, wherein the machine learning model has been trained differently, i.e. using a different loss function, than the model trained for localizing the abnormality. The model trained for "initial" localization of the abnormality is called the first model in the following and the model trained differently is called the second model in the following.
      The set of underlying voxels is then determined as the voxels which have a probability to belong to the abnormality that is comprised within a set minimal probability-value and a set maximal probability-value, wherein said probablity is determined by the second model and wherein the set minimal probablity-value and the set maximal probability-value may be different from the minimal probabitlyi-value and the maximal probability-value used during (initial) localization of the abnormality using the first model. In particular, the user can set at least one of the set minimal probability-value and/or the set maximal probability-value that is/are considered for determining the overall extension.
      Another example of such an automatic method to determine the underlying voxels is to determine that each voxel of the presentation belongs to the underlying voxels if the voxel is inside a signal range of values, starting at a minimal value and ending at a maximal value, wherein the user can modify either one or both of the minimal and maximal value.
   ∘ By indicating a corrected extension and/or corrected position in the presentation displayed in the image viewer.
      In other words, the user indicates the correct extension and/or correct position of a separate abnormality in the presentation displayed in the image viewer. Therefore, the user interface is configured to allow the user to indicate the corrected extension and/or corrected position of the abnormality related to an activated entry of the table in the presentation, and the user interface is configured to read out the corrected extension and/or corrected position.
   ∘ By selecting one of a plurality of probability segmentation maps that is generated in the step of screening the current presentation for the abnormality.
      The plurality of probability segmentation maps may be generated by the use of a plurality of models of the artificial intelligence and/or a model of a first artificial intelligence and a model of a second artificial intelligence as disclosed above, for example.
      Alternatively or in addition, the plurality of probability segmentation maps may be generated by using different minimal probability-values and/or maximal probability-values, wherein the minimal probability-values and the maximal probability-values are used to determine the range of probabilities indicating that a voxel with a given probability value belongs to the abnormality (probability within the range) or not (probability outside the range.
- The method comprises a step of generating at least one of the following lists for at least one of the abnormalities listed in the table.
   The at least one list may be displayed in the table, for example as a drop-down list or by tools configured to replace an item of the list shown in the table by another item of the list.
   ∘ A list comprising a plurality of position information indicating the position of the abnormality in the body portion.
   ∘ A list comprising a plurality of evolution information indicating the presence of the abnormality at a specific date, for example in the previous study.
      For example, the list may indicate the studies in which the abnormality related to an entry in the table were observed, for example by displaying or highlighting its date in the list or by displaying or highlighting its number in the list after ordering chronologically the studies and numbering them.
   ∘ A list comprising a plurality of contrast uptake information of the abnormality. In particular, contrast uptake information for different studies and/or different presentations, for example for the current study or presentation and the previous study or presentation, may be given in the list.
- A new entry for an abnormality not listed in the table can be generated in the table by the user and an extension and/or position of the abnormality can be defined by the user by indicating the extension and/or position in the presentation displayed image viewer.
   For example, the new entry may be generated by a one-click operation. For example, it may be generated by a corresponding button or by clicking in the presentation displayed in the image viewer on the position of the abnormality related to the new entry.
   In other words, the user interface may be configured to allow the user to indicate the position, and optionally extension, of the abnormality related to the new entry in the presentation displayed in the image viewer, and the user interface may be configured to read out the position, in particular to generate the position information indicating the position of the abnormality in the presentation, and optionally to read out the extension of the abnormality related to the new entry.
   Further information related to abnormality identified by the user may be generated in an automated manner, in particular any information that is generated for a separate abnormality that is identified in an automate manner.
- An entry listed in the table can be removed from the table by the user.
   For example, the entry may be removed by a one-click operation. For example, it may be removed by a corresponding button or by clicking in the presentation displayed in the image viewer on the abnormality related to the entry to be removed.

In summary and as mentioned above, the table may be editable by the user, wherein each entry of the table is editable independently of any other entry of the table and wherein the user has a plurality of opportunities to edit the table.

The method, in any embodiment disclosed, may comprise a step of compiling information for providing at least one file.

The information may be compiled and/or the file may be generated by use of links, operations etc.

A generated file comprises a time stamp related to the time the study was carried out and, for each abnormality that is observable in the study, a position information. The generated file might also comprise an evolution tag indicating whether there is a related abnormality in a study having an older time stamp than the time stamp related to the study of the generated file. The generated file might also comprise presentations of the studies and the abnormalities.

The presentations comprised in the generated file may be all presentations of the study or a selection thereof. The study may be taken according to a given procedure and/or stored in an independent format, such as the so-called DICOM format.

The abnormalities that are observable in the study may be the abnormalities identified in a step of screening and a step of identifying according to any embodiment disclosed plus the abnormalities added by a user and minus the abnormalities removed by a user. Adding and/or removing of abnormalities may be carried out in any embodiment disclosed, in particular in any embodiment disclosed that comprises the editable table.

In particular, the position information is an information suitable for determining whether there is a related abnormality in another study. In particular, the position information may be the position information indicating the position of the abnormality in a presentation or a position information derived thereof. The latter may be needed and/or beneficial because the generated file may concern a study comprising a plurality of presentations of the body portion.

Usually, a current file is generated and provided during each execution of the method if a new study, this means a study not yet considered in a previous execution of the method, is available. In other words, a new file is generated and provided whenever the method is executed on a new study. This also means that the "current file" generated and provided for the study preceding the new study becomes a (or rather the) "previous file" (and then the 2^{nd} last file etc.)..

The current file comprises presentations of the current study to which the current presentation belongs and the time stamp of the current study, a position information for each abnormality observable in the current study, and an evolution tag for each abnormality observable in the current study.

The previous file comprises presentations of a previous study to which the previous presentation belongs and the time stamp of the previous study, a position information for each abnormality observable in the previous study, and an evolution tag for each abnormality observable in the previous study.

The method, in any embodiment disclosed, may comprise a step of providing a radiological report, wherein the report comprises information given in or linked to the table.

The information given in the report may be any information given in or linked to the table, in particular at least one of the following information: The abnormalites identified in the current study, the position of each identified abnormality in the body portion, a hint whether the abnormality was identified in a previous study or not, the extend of each identified abnormality, the abnormalities identified in a previous study but not in the current study, the position and/or extend of the abnormalities identified in a previous study but not in the current study, a presentation of each abnormality identified in the current study, a presentation that is representative for the medical condition according to the current study, a summary list.

The report may be provided on the basis of the table shown in the user interface after request by the user, for example by pushing a table generation button of user interface.

The invention relates further to a system for supporting observation of a medical abnormality in or on a body portion displayed in a virtual medical presentation of the body portion.

The system may be configured or may have components configured to carry out any step of the method in any embodiment disclosed and/or to provide any opportunity to a user in any embodiment disclosed with respect to the method.

Likewise, the method in any embodiment disclosed may comprise any step related to any feature disclosed with respect to the system.

The system according to the invention comprises a communication unit, a controller and a user interface.
- The communication unit is configured to communicate with the controller and the user interface. Optionally, the communicatin unit may be configured to communicate with at least one of the imaging system used for generating the presentation or study to which it belongs and server/archive, in particular a server/archive on or in which the presentation or study is stored, such as a picture archiving communication system (PACS). The communication unit may be configured further to receive the presentation, in partiuclar any presentation or study processed by the system.
- The controller is configured for screening in an automated or semi-automated manner a virtual current presentation of a current study of the body portion for the abnormality, for identifying in the current presentation in an automated or semi-automated manner separate abnormalities of the abnormality for which the current presentation is screened, and for determining in an automated or semi-automated manner and for each identified abnormality a position information indicating the position of the identified abnormality in the current presentation.
   The controller may be configured for screening the current presentation for the abnormality according to any embodiment of the step of screening the current presentation for the abnormality disclosed with respect to the method.
   The controller may be configured for identifying separate abnormalities according to any embodiment of the step of identifying separate abnormalities disclosed with respect to the method.
   The controller may be configured for determining for each identified abnormality a position information indicating the position of the identified abnormality in the current presentatin according to any embodiment of the step of determining for each identified abnormality a position information disclosed with respect to the method.
   In particular, the position information is suitable for determining whether there is a related abnormality in another presentation or study and/or for generating a position information indicating the position of the abnormality in the body portion and/or for determining an extension, in particular a volume, of the abnormality, for example.
- The controller is configured further for generating in an automated manner a table. The table lists the identified abnormalities and, for each listed abnormality, a position information indicating the position of the abnormality in the body portion.
   The table may be a table according to any embodiment disclosed with repsect to the method.
   In particular, the table may be the editable table and/or active table and/or the table having entries linked to presentations according to any embodiment disclosed with respect to the method.
   The position information indicating the position of the abnormality in the body portion may be according to any position information indicating the position of the abnormality in the body portion disclosed with respect to the method, for example it may indicate its anatomic location.
- The user interface is configured for providing the table and a virtual presentation of the body portion comprised in the current study to a user, wherein the user interface comprises an image viewer configured for displaying the virtual presentation.
   The presentation provided together with the table to the user and the manner the presentation is provided to the user may be as disclosed with respect to any embodiment of the step of providing the table and a a virtual presentation of the body portion.
   The user interface and its image viewer may be according to any embodiment disclosed with respect to the method. In particular, it may comprise one or more control element according to any embodiment disclosed with respect to the method. In particular, the user interface may be configured for correcting, adding and/or removing entries in the table according to any embodiment disclosed with respect to the method. Therefore, the user interface may comprise the quantitative correction area described further below.
   The image viewer may be an image viewer according to any embodiment disclosed with respect to the method.
   In particular, the image viewer may be configured to display one presentation at once only.

In an embodiment, the system is a system for observing a change of the body portion over time, in particular a change with respect to an abnormality. Therefore, the system is configured for determining, for each identified abnormality, whether there is a related abnormality in a previous presentation of a previous study of the body portion.

This may be done by determining, using the position information indicating the position of the identified abnormality in the current presentation, whether there is an abnormality in the previous presentation having a position information indicating that the abnormality in the previous presentation corresponds to the abnormality identified in the current presentation.

Further, the table provided to the user indicates, for each abnormality listed, whether there is a related abnormality in the previous presentation or not. In particular, the table may comprise the evolution information according to any embodiment disclosed with respect to the method.

The system may be configured for determining, for each identified abnormality, whether there is a related abnormality in a previous presentation of the body portion according to any embodiment disclosed with respect to the step of determining for each identified abnormality whether there is a related abnormality in a previous presentation of the body portion.

The system may be configured for determining, for each identified abnormality, whether there is a related abnormality in a previous presentation of the body portion by the communication unit being configured to receive position information related to abnormalities observed in the previous presentation or previous study to which the previous presentation belongs and by the controller being configured to screen the position information related to the previous presentation or previous study for a position information corresponding to the position information of the abnormality identified in the current presentation.

In particular, the system may be configured for determining, for each identified abnormality, whether there is a related abnormality in a previous presentation of the body portion via the use of a current data set and a previous data set according to any embodiment disclosed with respect to the method.

In addition or alternatively, the system may be configured to set an evolution tag, in particular an evolution tag according to any embodiment disclosed with respect to the method.

In an embodiment, the system is configured and the user interface comprises one-click tools for allowing the user to at least one of:
- Replacing the presentation displayed in the image viewer with a replacement presentation that is a presentation of the same study as the displayed presentation. However, the replacement presentation is usually of another kind as the displayed presentation.
- Replacing the presentation displayed in the image viewer with a replacement presentation that is a presentation of the same kind as the displayed presentation. However, the replacement presentation is usually a presentation of another study than the displayed presentation.
- Toggling, this means switching back and forth, between displaying in the image viewer a first presentation and a second presentation, wherein the first presentation and the second presentation are presentations of the same study but usually of a different kind of presentation.
- Toggling, this means switching back and forth, between displaying in the image viewer a first presentation and a second presentation, wherein the first presentation and the second presentation are presentations of the same kind of presentation but usually of different studies.

The above-listed replacing and switching (toggling) may be according to any replacing and switching disclosed with respect to the method. In particular, the list of studies in any embodiment disclosed with respect to the method and the list of presentations in any embodiment disclosed with respect to the method may be used for replacing and/or switching. This also means that the user interface may have the first view and second view according to any embodiment disclosed with respect to the method and optionally any tools disclosed in the method with respect to replacing and switching.

In an embodiment of the system according to any embodiment disclosed, the user interface comprises a quantitative correction area. The quantitative correction area is configured for allowing the user to at least one of:
- Correcting the extension and/or position of an abnormality listed in the table. The correction according to any embodiment disclosed with respect to the method. In particular, the quantitative correction area may provide at least one of:
   ∘ A tool for modifying at least one of a minimal probability-value that is considered in the step of screening the current presentation for the abnormality and a maximal probability-value that is considered in the step of screening the current presentation for the abnormality.
   ∘ A tool for adding directly voxels or removing directly voxels form a set of voxels representing the abnormality listed in the table.
   ∘ An image viewer configured to indicate a corrected extension and/or corrected position in the presentation displayed in the image viewer.
   ∘ A tool for displaying different probability segmentation maps and for selecting the localization of the abnormality related to a selected probability segmentation map. In other words, the output of the step of screeing, and hence of the subsequent steps, may be influenced by selecting a probability segmentation map.
- Generating, in the table, a new entry for an abnormality not listed in the table and defining the extension and/or position of the abnormality by indicating the extension and/or position in the presentation displayed image viewer.
   In particular, the quantitative correction area comprises a button for adding an entry and/or the user interface is configure to receive information from the user by positioning a user-driven element on the user interface, in particular on the image viewer.
   The generation of a new entry may be according to any embodiment disclosed with respect to the method.
- Removing an entry listed in the table from the table.
   In particular, the quantitative correction area comprises a button for removing an entry and/or the user interface is configure to receive information from the user by positioning a user-driven element on the user interface, in particular on the image viewer.
   The removal of an entry may be according to any embodiment disclosed with respect to the method.

The invention concerns further a computer programm comprising instructions which, when the program is executed by a computer, cause the computer to carry out the method according any embodiment disclosed.

The invention concerns further a computer-readable medium having stored thereon instructions which, when executed by a computer, cause the computer to carry out the method for supporting observation of an abnormality according to any embodiment disclosed.

The invention concerns further a data carrier signal carrying instructions which, when executed by a computer, cause the computer to carry out the method for supporting observation of an abnormality according to any embodiment disclosed.

In particular, the invention concerns any reproducible computer-readable signal encoding the computer program that, when loaded and executed on a computer, causes the computer to carry out the method for supporting observation of an abnormality according to any embodiment disclosed.

The computer that is caused to carry out the method may be a computer or a computer network of the system in any embodiment disclosed.

The invention concerns further a method of manufacturing a non-transitory computer readable medium, comprising the step of storing, on the computer readable medium, computer-executable instructions which when executed by a processor of a computing system, cause the computing system to perform the method for supporting observation of an abnormality according to any embodiment disclosed.

### BRIEF DESCRIPTION OF THE DRAWINGS

Exemplarily embodiments of the invention are shown in the following figures. In the figures, the same reference symbol is used for identical or comparable elements. It shows:
- **Figure 1**: a flow chart of basic steps of a method according to the invention;
- **Figure 2**: a schematic representation of basic elements of a system according to the invention;
- **Figure 3**: a flow chart of an embodiment of the method that is suitable for observing a change of the body portion over time, in particular a change with respect to an abnormality; and
- **Figure 4**: an exemplary embodiment of the user interface.

### DETAILED DESCRIPTION OF THE EMBODIMENTS

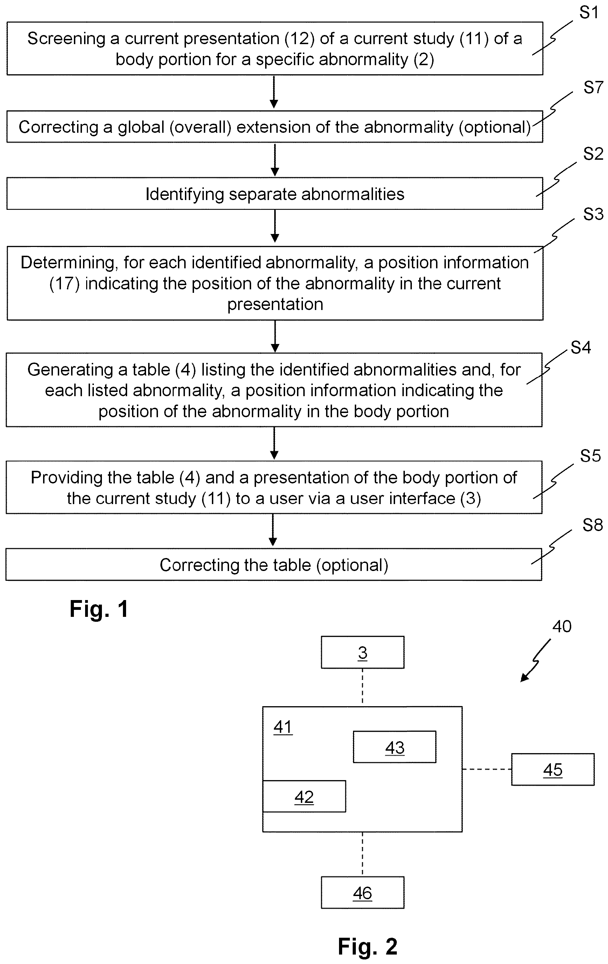

**Figure 1** shows a flow chart of basic steps of a method for supporting the observation of an abnormality 2 in or on a body portion displayed in a virtual medical presentation of the body portion. The basics steps, as well as most of the optional steps disclosed, aim to generate a table 4 listing the abnormalities 2, in particular the separate (individual) abnormalities of the same kind of abnormality, identified in the presentation or in the study to which the presentation belongs. Thereby, the table 4 comprises an entry, for example a row, for each identified abnormality. The entry comprises further information comprising a position information related to the abnormality to which the entry relates, in particular a position information indicating the position of the abnormality in the body portion.

Due to this purpose of the method, the method comprises the following basic steps:
- A step S1 of screening a current presentation of a current study of the body portion for a specific abnormality 2, for example a lesion related to multiple sclerosis (MS).
- A step S2 of identifying separate abnormalities of the abnormality for which the current presentation is screened.
- A step S3 of determining, for each identified abnormality 2 a position information indicating the position of the abnormality in the current presentation.
- A step S4 of generating a table 4 wherein the table 4 comprises for each identified abnormality 2 an entry 101, this means the table 4 lists the identified abnormalities 2, and wherein the table comprises a position information indicating the position of the abnormality in the body portion for each listed abnormality.
   In an embodiment, the step S4 of generating a table is an automated step that is started automatically after the step S3 of determining, for each abnormality identified, a position information indicating the position of the abnormality in the current presentation.
- A step S5 of providing the table 4 and a virtual presentation of the body portion, said virtual presentation being comprised in the current study, to the user via a user interface 3.
   Therefore, the user interface 3 comprises an image viewer 5.
   The step S5 of providing the table 4 and the presentation is usually an automated step that is executed automatically after generation of the table 4.
   The presentation comprised in the current study and provided to the user is usually the current presentation based on which the table 4 was generated, this means the presentation that was screened for the abnormality, or a presentation of the current study that is representative for the present medical case, for example by showing all or most identified abnormalities in a clear manner.

The method may comprise further the optional step S8 of correcting the table 4. This is in particular the case if a report is to be generated in an automated manner from the table and/or if the present study is one of a plurality of studies for observing the abnormality.

For example, the step S8 of correcting the table 4 may comprise at least one of adding an entry in the table 4, removing an entry in the table 4, and correcting an entry in the table, in particular the position and/or extend of the abnormality related to said entry. Details of adding an entry, removing an entry and correcting an entry are given above.

The steps of screening, identifying and determining a position information are usually carried out by an algorithm configured to carry out these steps in a subsequent and automated manner, except an optional initialization by the user and an optional step S7 of correcting an amount of voxels determined by the algorithm as showing the abnormality.

The optional step S7 of correcting comprises for example selecting one of a plurality of probability segmentation maps calculated for the current presentation and/or modifying a minimal probability-value and/or a maximal probability value, wherein the minimal and maximal probability-values define a range of probability values, wherein a voxel of the current presentation is considered to show the abnormality if a calculated probability value of the voxel is within said range.

The current presentation that is screened may be determined by an automated method, such as by a set of rules defined by the user or by an artificial intelligence, such as a model trained to recognize a specific contrast, such as a MRI FLuid Attenuated Inversion Recovery (FLAIR) contrast. Alternatively, it may be determined by the user, for example by selecting a presentation from the presentations of the current study.

In the embodiment shown, identification of the separate abnormalities is done by screening an overall (global) extension of the abnormality for non-continuous sub-extensions. The overall (global) extension of the abnormality is the outcome of the step S1 of screening the current presentation for the abnormality using a model of an artificial intelligence trained for segmenting the current presentation in voxels (or pixels as the case may be) comprising the abnormality and in voxels (pixels) not comprising the abnormality.

The position information may be a position information indicating the position of the identified abnormality in the current presentation, for example the voxels (or pixels) related to the abnormality. The position information indicating the position of the identified abnormality in the body portion is usually derived from the position information indicating the position of the identified abnormality in the current presentation that may be a direct outcome of the step S3 of determining a position information.

The position information indicating the position of the identified abnormality in the current presentation may be used further in at least one of the following optional steps:
- A step S6 of determining for each identified abnormality whether there is a related abnormality in a previous presentation, in particular a previous study, of the body portion of the specific patient.
- A step of highlighting the abnormality 2 in a displayed presentation 6 of the body portion.
- A step of determining an extent, for example a volume if the current presentation is a 3D representation of the body portion.
- The step S8 of correcting the table, in particular the position and/or extend of the identified abnormality having said position information indicating its position in the current presentation.

**Figure 2** shows a schematic representation of basic elements of a system 40 for supporting the observation of a medical abnormality in or on a body portion displayed in a virtual medical presentation of the body portion.

The system 40 comprises the user interface 3, a communication unit 41, a controller 46 and a memory 45.

The controller 46 of the system is configured to carry out the steps of the method, in particular the steps of the method that are resource-intense and not directly related to the communication of the system or the interaction with the user.

The communication unit 41 comprises a communication module 42 for receiving data, in particular presentations and/or studies and information related to the presentations and/or studies, from an imaging system and/or from an image archive, such as a PACS.

The communication unit 41 comprises further a controler 43 configured to control the communication unit 41.

The controller 46 may be the controller 43 of the communication unit 41. In addition or alternatively, the memory 45 may be a mermory of the communicatin unit 41.

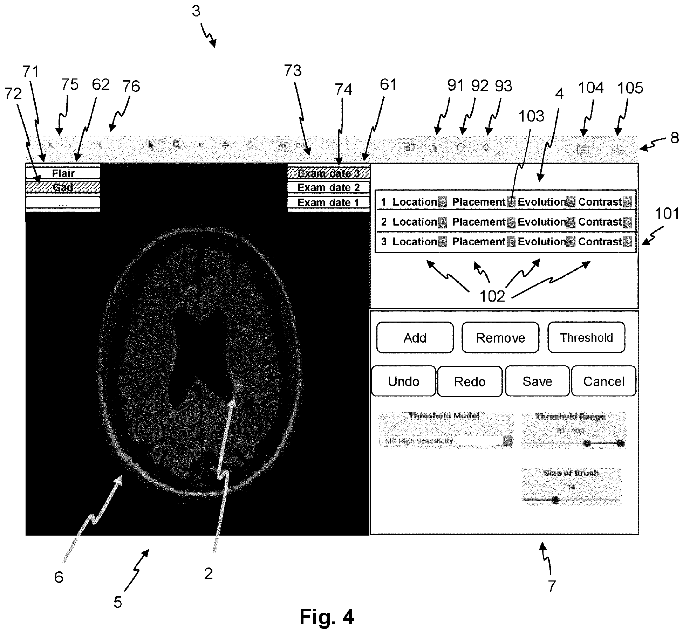

The user interface is as disclosed with respect to figure 4, for example.

The system and its elements are configured to carry out the method according to any embodiment disclosed.

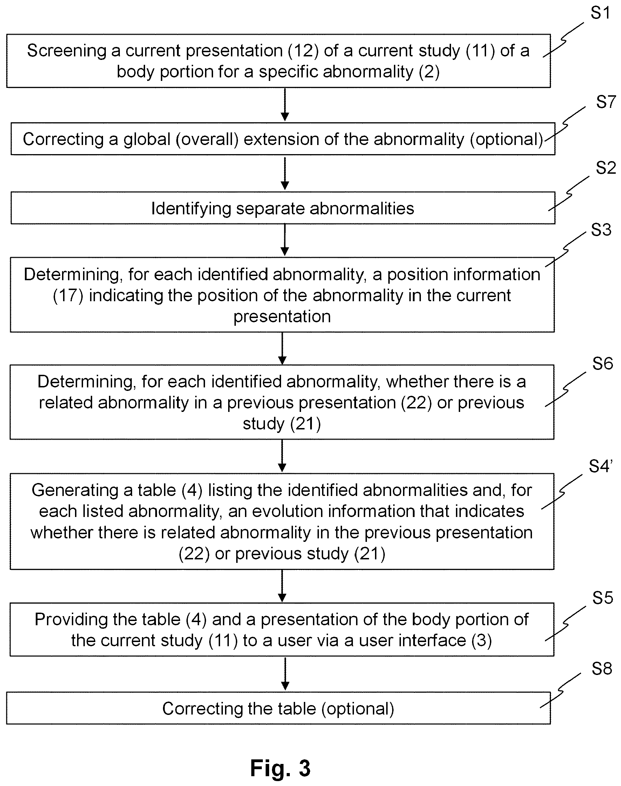

**Figure 3** shows a flow chart of an embodiment of the method that is suitable for observing a change of the body portion over time, in particular a change with respect to an abnormality.

The method according to figure 3 comprises the steps disclosed with respect to figure 1 and a step S6 of determining, for each abnormality identified in the current presentation, or - more general - in the current study, whether there is a related abnormality in a presentation that was taken some time, for example some days, weeks or months (depending on the monitoring cycle used for monitoring the specific abnormality) before the current presentation or - more general - whether there is a related abnormality in a study that was taken some time, for example some days, weeks or month (depending on the monitoring cycle used for monitoring the specific abnormality) before the current presentation was taken.

A presentation that was taken some time before the current presentation is called a previous presentation.

A study that was taken some time before the current study is called a previous study.

In the embodiment shown in figure 3, the step S6 of determining related abnormalities in a previous presentation or previous study is carried out, for example in an automated manner except a potential initiation by the user, after the step S3 of determining for each identified abnormality a position information indicating the position of the abnormality in the previous presentation (study, as the case may be). However, the step S6 of determining related abnormalities in the previous presentation or the previous study may be carried out after the step S8 of correcting the table, in particular the position and/or extend of the identified abnormalities, or it may be carried out again after the step S8 of correcting.

Further, the method according to figure 3 comprises a modified step S4' of generating a table 4 in which a table 4 is generated that comprises further an evolution information for each abnormality listed in the table. It goes without saying that the modified step S4 of generating a table 4 is carried out (again, as the case may be) after the step S6 of determining related abnormalities in a previous presentation or previous study

In an embodiment, the evolution information consists of the information whether the abnormality to which the entry of the table relates was present in the previous presentation or the previous study or not.

In an embodiment, the evolution information comprises the information whether the abnormality to which the entry of the table relates was present in the previous presentation or the previous study or not. In this embodiment, the evolution information may comprise further information such as a change in extent and/or volume, a change in position of the abnormality, a direction of growing and/or a direction of shrinkage, for example.

**Figure 4** shows an exemplary embodiment of the user interface 3 comprising as a central element the table 4 and a plurality of optional features configured to activate or carry out optional functions.

In the embodiment shown, the table 4 is an editable table with an entry 101 for each abnormality identified in an automated or semi-automated manner, and optionally corrected by the user, by a method as disclosed with respect to figures 1 and 3 for example.

Each entry 101 comprises entry characteristics 102 that give further information concerning the abnormality to which the entry belongs. The position of the abnormality in the body portion (called location in figure 6), specific information concerning a derived property of the position of the abnormality in the body portion, (called placement in figure 6), the information whether the abnormality is new or present in the previous study (called evolution in figure 6) and information related to the contrast uptake of the abnormality (called contrast in figure 6), which is an important information for example in inflammatory and oncologic diseases.

The table 4 shown is a control element by itself by being configured to amend an entry characteristic 102 and to show a different presentation 6 in the image viewer 5 by the entry characteristics being realized or linked to control elements 103.

The user interface 3 according to figure 6 comprises further the following elements:
- The image viewer 5 for displaying a presentation 6, in particular a single presentation.
- The quantitative correction area 7 for correcting the table 4 and/or parameters that have an effect on algorithms used in the automated steps, in particular the step S2 of screening the current presentation and the step S8 of correcting the table.
- A list 71 of presentations indicating available alternative kinds of presentations for the presentation 6 displayed in the image viewer 5.
   In the embodiment shown, the list 71 of presentations is configured as a control element by being able to replace the displayed presentation 6 by a corresponding presentation of another kind, for example by being taken with another contrast or by being generated differently from a row acquisition, by clicking on said other kind in the list. The currently selected kind of presentation 72 is highlighted in the list 71 of presentations.
- A list 73 of studies indicating available exam dates, for example for the presentation 6 displayed in the image viewer 5.
   In the embodiment shown, the list 73 of studies is configured as a control element by being able to replace the displayed presentation 6 by a corresponding presentation taken at another date by clicking on said other date in the list. The currently selected study 74 is highlighted in the list 73 of studies.
- A first view 61 for the list 73 of studies and a second view 62 for the list 71 of presentations
- A toolbar 8 comprising various control elements, such as:
   ∘ A toolbar switch 75 for switching the currently displayed kind of presentation, in particular for navigating, this means move up and down, in the list 71 of presentations.
   ∘ A toolbar switch 76 for switching the currently displayed exam date, in particular for navigating, this means move up and down, in the list 73 of studies.
   ∘ A table generation button 91 configured to generate a new table 4 or to update a table 4 shown in the user interface 3. The new or updated table may consider the displayed presentation 6 and/or corrections made to the shown table and the abnormalities linked to its entries.
   ∘ An abnormality display button 92 configured to show in the displayed presentation 6 a marking around the abnormalities listed in the table 4 with a special marking around the currently selected abnormality in the table 4.
   ∘ An abnormality extension button 93 configured to show an extension of an abnormality selected in the table, and to bring the user interface 3 in a mode in which the user can correct an extension of an abnormality 2 shown in the displayed presentation 6.
   ∘ A report generation button 104 configured to initiate automatic report generation from the table 4 shown on the user interface 3. The report generation button 104 initiates further displaying of the generated report on the user interface in an editable manner.
   ∘ A report saving button 105 configured to save the report shown on the user interface 3.

### Reference signs

- 2.: Abnormality
- 3.: User Interface
- 4.: Table
- 5.: Image viewer
- 6.: Displayed presentation
- 7.: Quantitative correction area
- 8.: Toolbar
- 40.: System
- 41.: Communication unit
- 42.: Communication module
- 43.: Controller of communication unit
- 45.: Memory
- 46.: Controller
- 61.: First view
- 62.: Second view
- 71.: List of presentations
- 72.: Currently selected item of the list of presentations
- 73.: List of studies
- 74.: Currently selected item of the list of studies
- 75.: Toolbar switch for switching currently displayed kind of presentation
- 76.: Toolbar switch for switching current displayed study
- 91.: Table generation button
- 92.: Abnormality display button
- 93.: Abnormality extension button
- 101.: Entry in table
- 102.: Entry characteristic
- 103.: Control element
- 104.: Report generation button
- 105.: Report saving button
- S1: Screening a presentation of a current study (current presentation) for an abnormality
- S2: Identifying separate abnormalities
- S3: Determining, for each identified abnormality, a position information indicating the position of the identified abnormality in the current presentation
- S4: Generating a table listing the identified abnormalities and, for each identified abnormality, a position information indicating the position of the abnormality in the body portion
- S4': Generating a table comprising further evolution information
- S5: Providing the table and a presentation of the current study to a user
- S6: Determining, for each identified abnormality, if there is a related abnormality in a previous presentation
- S7: Correcting a global (overall) the extension of the abnormality
- S8: Correcting the table, in particular the extend and/or position of an identified abnormality by the user

## Claims

1. A computer-implemented method for supporting observation of a medical abnormality (2) in or on a body portion displayed in a virtual medical presentation of the body portion, the method comprises:
• A step (S1) of screening a virtual current presentation of a current study of the body portion for the abnormality (2), wherein the step (S1) of screening is an automated or semi-automated step;
• A step (S2) of identifying separate abnormalities in the current presentation, wherein the step (S2) of identifying is an automated or semi-automated step;
• A step (S3) of determining, for each identified abnormality, a position information indicating the position of the identified abnormality in the current presentation, wherein the step (S3) of determining is an automated or semi-automated step;
• A step (S4, S4') of generating, in an automated manner, a table (4) listing the identified abnormalities (2) and, for each listed abnormality, a position information indicating the position of the abnormality in the body portion;
• A step (S5) of providing the table and a virtual presentation of the body portion comprised in the current study to a user via a user interface (3), wherein the user interface comprises an image viewer (5) and the virtrual presentation is displayed in the image viewer.

2. The method according to claim 1, comprising a step (S6) of determining, for each identified abnormality (2), whether there is a related abnormality in a virtual previous presentation of a previous study of the body portion by determining, using the position information indicating the position of the identified abnormality in the current presentation, whether there is an abnormality in the previous presentation having a position information indicating that the abnormality in the previous presentation corresponds to the abnormality identified in the current presentation, wherein the table (4) indicates whether there is a related abnormality in the previous presentation or not.

3. The method according to claim 2, wherein the step (S6) of determining, for each identified abnormality (2), whether there is a related abnormality in a virtual previous presentation of the body portion comprises:
• A step of generating a current data set comprising, for each identified abnormality, a current data element, wherein the current data element comprises the position information indicating the position of the identified abnormality in the current presentation, wherein the step of generating a current data set is an automated step;
• A step of receiving, in an automated manner, a previous data set of the previous presentation of the body portion, wherein the previous data set comprises, for each abnormality identified in the previous presentation, a previous data element, wherein the previous data element comprises a position information indicating the position of the abnormality identified in the previous presentation;
• A step of screening, for each current data element, the previous data set for a previous data element comprising the abnormality at a position in the previous presentation that corresponds to the position of the abnormality according to the current data element, wherein the step of screening the previous data set is an automated step.

4. The method according to claim 3, comprising a step of setting an evolution tag for each current data element for which a previous data element comprising the abnormality at a position in the previous presentation that corresponds to the position of the abnormality according to the current data element was found, wherein the step of setting an evolution tag is an automated step.

5. The method according to one of claims 1-4, comprising:
• A step of providing a list of studies (73) comprising a first item that is characteristic for the current study and a second item that is characteristic for a previous study;
• A step of providing a list of presentations comprising at least one item that is characteristic for a kind of presentation;
• A step of assigning the current study to the item of the list of studies that is characteristic for the current study and assigning the previous study to the item of the list of studies that is characteristic for the previous study;
• A step of assigning a presentation of the current study to the item of the at least one item of the list of presentations that is characteristic for the presentation of the current study and a presentation of the previous study to the item of the at least one item of the list of presentations that is characteristic for the presentation of the previous study;
• A step of providing to the user via the user interface (3) the list of studies or an adapted list of studies and the list of presentations or an adapted list of presentations,
wherein the user interface comprises a first view (61) for the list of studies or the adapted list of studies and a second view (62) for the list of presentations or the adapted list of presentations;
• A step of selecting a presentation to be displayed in the image viewer by selecting an item of the list shown in the first view and by selecting an item of the list shown in the second view.

6. The method according to claim 5, wherein the user interface (3) comprises tools allowing the user to at least one of:
• Replacing the presentation (6) displayed in the image viewer (5) with a replacement presentation that corresponds to the presentation assigned to the item arranged in the list shown in the second view (62) precedingly or subsequently to the item to which the presentation displayed in the image viewer is assigned;
• Replacing the presentation (6) displayed in the image viewer (5) with a replacement presentation that belongs to the study assigned to the item arranged in the list shown in the first view (61) precedingly or subsequently to the item to which the study is assigned to which the presentation displayed in the image viewer belongs;
• Switching back and forth between displaying in the image viewer (5) a first presentation and a second presentation, wherein the first presentation is a presentation assigned to a first item in the list shown in the second view (62) and the second presentation is a presentation assigned to a second item in the list shown in the second view;
• Switching back and forth between displaying in the image viewer (5) a first presentation and a second presentation, wherein the first presentation is a presentation of the study assigned to a first item in the list shown in the first view (61) and the second presentation is a presentation of the study assigned to a second item in the list shown in the first view.

7. The method according to one of claims 1-6, wherein the step (S1) of screening the current presentation for the abnormality (2), comprises a step of running a model of an artificial intelligence, wherein the model is trained for assigning a probability to each voxel a presentation of the body portion that the voxel belongs to the abnormality.

8. The method according to claim 7, wherein the step of running a model of an artificial intelligence comprises running a plurality of models of the artificial intelligence and/or a model of a first artificial intelligence and a model of a second artificial intelligence, wherein the models run are trained for assigning a probability to each voxel of a presentation of the body portion that the voxel belongs to the abnormality, wherein, for each model run, a probability segmentation map is generated, and wherein the user can select a generated probability segmentation map.

9. The method according to one of claims 1-8, wherein at least one of the following applys:
• The method comprises a step (S8) of correcting by the user the extend and/or position of an identified abnormality by at least one of the following:
i. by modifying at least one of a minimal probability-value that is considered in the step (S1) of screening the current presentation for the abnormality and a maximal probability-value that is considered in the step (S1) of screening the current presentation for the abnormality;
ii. by directly adding voxels to or removing voxels from a set of voxels representing an abnormality listed in the table;
iii. by indicating a corrected extension and/or corrected position in the presentation displayed in the image viewer (5);
iv. by selecting one of a plurality of probability segmentation maps that is generated in the step (S1) of screening the current presentation for the abnormality.
• The method comprises a step of generating at least one of the following lists for at least one abnormality listed in the table:
i. A list comprising a plurality of position information indicating the position of the abnormality (2) in the body portion;
ii. A list comprising a plurality of evolution information indicating the presence of the abnormality at a specific date;
iii. A list comprising a plurality of contrast uptake information of the abnormality;
• A new entry for an abnormality not listed in the table (4) can be generated in the table by the user and an extension and/or position of the abnormality can be defined by the user by indicating the extension and/or position in the presentation displayed in the image viewer (5).
• An entry for an abnormality can be removed from the table (4).

10. The method according to one of claims 1-9, comprising a step of providing a radiological report, wherein the report comprises information given in or linked to the table (4).

11. The method according to claim 10, wherein the report comprises at least one of the following information: The abnormalites identified in the current study, the position of each identified abnormality in the body portion, a hint whether the abnormality was identified in a previous study or not, the extend of each identified abnormality, the abnormalities identified in a previous study but not in the current study, the position and/or extend of the abnormalities identified in a previous study but not in the current study, a presentation of each abnormality identified in the current study, a presentation that is representative for the medical condition according to the current study, a summary list.

12. A system (40) for supporting observation of a medical abnormality (2) in or on a body portion displayed in a virtual medical presentation of the body portion, the system comprises a communication unit (41), a controller (46) and a user interface (3), wherein:
• The communication unit is configured to communicate with the controller and the user interface;
• The controller is configured for screening in an automated or semi-automated manner a virtual current presentation of a current study of the body portion for the abnormality (2), for identifying in an automated or semi-automated manner separate abnormalities in the current presentation, and for determining in an automated or semi-automated manner and for each identified abnormality a position information indicating the position of the identified abnormality in the current presentation;
• The controller is configured for generating in an automated manner a table (4) listing the identified abnormalities (2) and, for each listed abnormality, a position information indicating the position of the abnormality in the body portion;
• The user interface is configured for providing the table and a virtual presentation of the body portion comprised in the current study to a user, wherein the user interface comprises an image viewer (5) configured for displaying the virtual presentation.

13. The system (40) according to claim 12, wherein the system is configured for determining, for each identified abnormality (2), whether there is a related abnormality in a virtual previous presentation of a previous study of the body portion by determining, using the position information indicating the position of the identified abnormality in the current presentation, whether there is an abnormality in the previous presentation having a position information indicating that the abnormality in the previous presentation corresponds to the abnormality identified in the current presentation, and wherein the table (4) indicates whether there is a related abnormality in the previous presentation or not.

14. The system (40) according to claim 12 or 13, wherein the system is configured and the user interface (3) comprises one-click tools for allowing the user to at least one of:
• Initiating a replacement of a presentation (6) displayed in the image viewer (5) with a replacement presentation, wherein the displayed presentation (6) and the replacement presentation are presentations of the same study and of a different kind of presentation.
• Initiating a replacement of a presentation (6) displayed in the image viewer (5) with a replacement presentation, wherein the displayed presentation (6) and the replacement presentationare presentations of the same kind of presentation and of different studies.
• Switching back and forth between displaying in the image viewer (5) a first presentation and a second presentation, wherein the first presentation and the second presentation belong to the same study and are of a different kind of presentation.
• Switching back and forth between displaying in the image viewer (5) a first presentation and a second presentation, wherein the first presentation and the second presentation are of the same kind of presentatin and belong to different studies.

15. The system (40) according to one of claims 12-14, wherein the user interface (3) comprises a quantitative correction area (7) allowing the user to at least one of:
• Correcting the extend and/or position of an abnormality listed in the table (4);
• Generating, in the table (4), a new entry for an abnormality not listed in the table (4) and defining the extension and/or position of the abnormality by indicating the extension and/or position in the presentation displayed image viewer (5).
• Removing an entry for an abnormality from the table (4).

16. A computer program comprising instructions which, when the program is executed by a computer, cause the computer to execute the steps of the method according to one of the claims 1-11.

17. A reproducible computer-readable signal encoding the computer program according to claim 16.
